# EUROPEAN PATENT SPECIFICATION

(11) **EP 2 063 810 B1**
(45) Date of publication and mention of the grant of the patent: **06.06.2012**
(21) Application number: 07814774.1
(22) Date of filing: 10.09.2007
(51) Int. Cl.: A61F 2/92

(54) **BIOACTIVE AGENT-CONTAINING IMPLANTABLE RINGS**
EINEN BIOLOGISCHEN WIRKSTOFF ENTHALTENDE IMPLANTIERBARE RINGE
Anneaux implantables contenant un agent bioactif

(30) Priority: 08.09.2006 US 843022 P; 07.09.2007 US 852212
(43) Date of publication of application: 03.06.2009
(73) Proprietor: Abbott Cardiovascular Systems Inc., Santa Clara, CA 95054-2807 (US)
(72) Inventor: KRAMER-BROWN, Pamela A., San Jose, California 95135 (US); LUDWIG, Florian N., 6030 Ebikon, Luzern, (CH)
(74) Representative: ZBM Patents
(86) International application number: PCT/US2007/078035
(87) International publication number: WO 2008/031097

(56) References cited:
- WO-A-02/055123
- US-A1- 2003 069 593
- US-A1- 2005 163 821
- US-A1- 2005 203 613
- US-B1- 6 416 549

## Description

### FIELD OF THE INVENTION

The present invention is directed to a bioactive agent-containing implantable rings and methods of using for the treatment of disease.

### BACKGROUND OF THE INVENTION

The traditional method of administering therapeutic agents to treat diseases of the internal organs and vasculature has been by systemic or local delivery. Systemic delivery involves administering a therapeutic agent at a discrete location followed by the agent migrating throughout the patient's body including, of course, to the afflicted organ or area of the vasculature. Systemic delivery is carried out primarily in two ways: introduction of the therapeutic agent into the digestive tract (enteral administration) or into the vascular system (parenteral administration), either directly such as injection into a vein or an artery or indirectly such as injection into a muscle or into the bone marrow. Delivery by each of these routes is strongly influenced by the so-called ADMET factors: absorption, distribution, metabolism, excretion and toxicity. For enteric administration, such factors as a compound's solubility, its stability in the acidic environs of the stomach and its ability to permeate the intestinal wall all affect the extent to which the drug is absorbed and therefore its bioavailability. For parenteral delivery factors such as enzymatic degradation, the lipophilic/hydrophilic partitioning coefficient and protein binding will affect the bioavailability of an agent.

At the other end of the spectrum is local delivery, which comprises administering the therapeutic agent directly to the afflicted site. The ADMET factors tend to be less important than with systemic administration since the agent is being administered essentially directly to the treatment site. Thus, the initial dose can be at or very close to the therapeutically effective amount. With time, some of the locally delivered therapeutic agent may diffuse over a wider region but such is not the intent of localized delivery and the concentration of the diffused agent will ordinarily be sub-therapeutic, i.e., too low to have a therapeutic effect. Since localized delivery targets only the desired treatment site, it is possible that some of the causal factors of the disease that have spread to as yet non-afflicted regions of the organ at the periphery of the afflicted region may not undergo sufficient treatment, resulting in reoccurrence of the disease.

What would be beneficial are devices and methods that can be used to treat a disease such that the ADMET factors are of reduced significance as in the case of local delivery while at the same time some of the broad coverage afforded by systemic delivery is maintained. The current invention provides such devices and methods.

US 6,416,549 discloses an antithrombogenic annuloplasty ring. At least some portion of the annuloplasty ring has incorporated into it or onto its structure one or more anithrombogenic agents or materials.

US 2005/0203613 discloses an implantable stent comprising a tubular member having an interior surface and an exterior surface with a region of at least one of the surfaces being hydrophobic. The stent may furthermore include a medicinal substance that adheres to exposed portions until the hydrophobicity of the region is altered.

US 2005/0163821 discloses an open-ring stent comprising a base member and a plurality of ring members secured to the base member. It furthermore discloses a stent with a coating, such as a drug-containing collagen layer.

### SUMMARY OF THE INVENTION

The present invention relates to an implantable medical device that includes an open ring-shaped device body having a first and second end and one or more bioactive agents adhered to a surface of, integrated into the structure of or disposed within pores in the device body and a tether, wherein the open ring-shaped device encircles the tether.

In one aspect, the first and second ends of the open ring-shaped device body overlap each other. In another aspect, the first and second ends of the open ring-shaped device body do not overlap each other.

In various aspects, the device body comprises a material selected from a group that includes nitinol, a biodegradable material, a polymeric material and any combination thereof.

In various aspects, the device body comprises a polymeric material and a nitinol core.

In various aspects, the implantable medical device further includes two or more open ring-shaped device bodies.

In various aspects, the implantable medical device further includes a degradable casing in which the open ring-shaped device body is contained in a compressed state. The casing can degrade within 10 seconds to 10 minutes.

In various aspects, the open ring-shaped device body abuts a spherical member coupled to the distal end of the tether. In one embodiment, the spherical member is biodegradable.

In various aspects, the implantable medical device further includes a plurality of tethers wherein one open ring-shaped device encircles each tether and abuts a spherical member. In various embodiments, the implantable medical device includes two or more open-ringed shaped devices encircling at least one tether. In other embodiments, the two or more open-ringed shaped devices each individually abut a spherical member coupled to the tether.

There is further disclosed a method for treating or preventing a disease. The method involves providing an implantable medical device of the invention, wherein the device is in a compressed state and positioned at the distal end of a catheter, inserting the catheter into a blood vessel of a patient upstream of a disease site locale and deploying the device into the bloodstream of the patient, wherein the device expands to the circumference of the blood vessel in which it is positioned.

In various aspects, deploying the device involves using a plunger having a longitudinally-oriented cylindrical member on which the compressed implantable medical device is positioned.

In various aspects, the disease to be treated is a vascular disease. When a vascular disease is to be treated it can be atherosclerosis, restenosis, vulnerable plaque or peripheral arterial disease.

In various aspects, the disease to be treated is selected from a group that includes cancer, coronary artery disease, kidney disease, liver disease, respiratory disease, infectious diseases and autoimmune diseases.

The disclosure further relates to a method for treating or preventing a disease. The method involves providing an implantable medical device according to the invention wherein the device is positioned in the distal end of a catheter, inserting the catheter into a blood vessel of a patient upstream of a disease site locale, deploying the device into the bloodstream of the patient such that the device is allowed to move in the direction of blood flow to be positioned at or near a disease site locale while the proximal end of the tether remains operatively coupled to the catheter and retrieving the tether.

In various aspects, the disease to be treated is a vascular disease. When a vascular disease is to be treated it can be atherosclerosis, restenosis, vulnerable plaque or peripheral arterial disease.

In various aspects, the disease to be treated is selected from a group that includes cancer, coronary artery disease, kidney disease, liver disease, respiratory disease, infectious diseases and autoimmune diseases.

### BRIEF DESCRIPTION OF THE DRAWINGS

Figures 1A-B depict a cross-sectional view of a blood vessel in which implantable medical devices of the invention are positioned. Figure 1A depicts a device positioned in a blood vessel in which the ends of an open ring-shaped device overlap each other. Figure 1B depicts a device positioned in a blood vessel in which the ends of the open ring-shaped device do not overlap each other.
Figure 2 depicts a cross-sectional view of a blood vessel showing a medical device in a compressed state in a catheter where the medical device is positioned on a cylindrical member of a plunger. A fully deployed medical device in the blood vessel is also shown.
Figure 3 illustrates a vascular network showing a device of the invention abutting a spherical member, both of which are contained within a degradable casing. A tether running from the spherical member through the device and to a catheter is also shown.

### DETAILED DESCRIPTION

### DEFINITIONS:

Use of the singular herein includes the plural and visa versa unless expressly stated to be otherwise. That is, "a" and "the" refer to one or more of whatever the word modifies. For example, "a bioactive agent" includes one such agent, two such agents, etc.

As used herein, "implantable medical device" refers to any type of appliance that is totally or partly introduced, surgically or medically, into a patient's body or by medical intervention into a natural orifice, and which is intended to remain there after the procedure. The duration of implantation may be essentially permanent, i.e., intended to remain in place for the remaining lifespan of the patient; until the device biodegrades; or until it is physically removed. At present, a preferred implantable medical device for use with this invention includes a open ring-shaped device body that can be employed for the regional or localized delivery of bioactive agents to specific treatment sites or areas in a patient's body.

As used herein, "device body" refers to a fully formed implantable medical device with an outer surface to which no coating or layer of material different from that of which the device itself is manufactured has been applied.

As used herein, "open ring-shaped device body" refers to generally circular shaped device body in which the ends of the circle are not connected. Rings in which the ends overlap or do not overlap are encompassed by the present invention. The terms "open ring-shaped device body", "device body" and "ring" and will be used interchangeably (unless the context shows otherwise).

As used herein, "adhered to the surface of" means the bioactive agent is covalently or non-covalently attached to the outer surface of the device body.

As used herein, "integrated into the structure of" means the bioactive agent is encapsulated in the material forming the device body.

As used herein, "disposed within pores" means the bioactive agent is located within pores, or holes, that are part of the device body.

As used herein, "tether" refers to any connecting mechanism, e.g., a string, that is flexible and capable of being coupled, either directly or indirectly to a component of the invention.

As used herein, "operatively coupled" refers to the attachment of a tether to the biodegradable spherical member, the biodegradable casing or the device body of the invention through either direct or indirect means. For example, the tether can be directly attached to the surface of the casing or the spherical member by a portion of the tether itself. Alternatively, the device body can encircle the tether, in which case it would be indirectly attached to the tether.

As used herein, "casing" refers to a material that completely encases a device body and optionally a spherical member of the invention. The casing will be readily biodegradable or biosoluble such that once the device body is positioned at a target site in the vasculature it will degrade or dissolve within a few seconds to a few minutes to release the device body within. Preferably, a casing will degrade within 10 seconds to 10 minutes after deployment *in vivo*. Examples of biodegradable and biosoluble materials include polyethylene glycol, polyvinyl pyrrolidone, polysaccharides, tyrosine-based polycarbonates and amphiphilic block copolymers such as poly ethylene glycol-poly lactic acid. Other suitable biodegradable materials are known to those skilled in the art.

As used herein, "biodegradable" as well as degraded, eroded, and absorbed, are used interchangeably (unless the context shows otherwise) and refer to materials that are capable of being degraded or absorbed when exposed to bodily fluids such as blood, and components thereof such as enzymes, and that can be gradually resorbed, absorbed, and/or eliminated by the body.

As used herein, "spherical member" refers to a generally round end piece attached to the distal end of the tether and on which the ring-shaped device body can be positioned during deployment. It can be biodegradable, biosoluble or biostable. Examples of biodegradable, biosoluble and biostable materials are described below, and others are known to those skilled in the art.

As used herein, "patient" refers to any organism that can benefit from the administration of a bioactive agent. In particular, patient refers to a mammal such as a cat, dog, horse, cow, pig, sheep, rabbit, goat or a human being.

As used herein, "treating" refers to the administration of a therapeutically effective amount of a bioactive agent to a patient known or suspected to be suffering from a disease.

As used herein, "therapeutically effective amount" refers to the amount of bioactive agent that has a beneficial effect, which may be curative or palliative, on the health and well-being of a patient with regard to a disease with which the patient is known or suspected to be afflicted. A therapeutically effective amount may be administered as a single bolus, as intermittent bolus charges, as short, medium or long term sustained release formulations or as any combination of these.

As used herein, "known" to be afflicted with a disease refers first to a condition that is relatively readily observable and or diagnosable. An example, without limitation, of such a disease is atherosclerosis, which is a discrete narrowing of a patient's arteries. Restenosis, on the other hand, while in its latter stages, like atherosclerosis, is relatively readily diagnosable or directly observable, may not be so in its nascent stage. Thus, a patient may be "suspected" of being afflicted or of being susceptible to affliction with restenosis at some time subsequent to a surgical procedure to treat an atherosclerotic lesion.

As used herein, "bioactive agent" can be used interchangeably with therapeutic agent. A bioactive agent of the invention can be an anti-restenosis agent, an antiproliferative agent, an anti-inflammatory agent, an antineoplastic, an antimitotic, an antiplatelet, an anticoagulant, an antifibrin, an antithrombin, a cytostatic agent, an antibiotic, an anti-allergic agent, an anti-enzymatic agent, an angiogenic agent, a cytoprotective agent, a cardioprotective agent, a proliferative agent, an ABC A1 agonist, an antioxidant, or any combination thereof.

Examples of antiproliferative agents include, without limitation, actinomycins, taxol, docetaxel, paclitaxel, rapamycin, 40-*O*-(3-hydroxy)propyl-rapamycin, 40-O-[2-(2-hydroxy)ethoxy]ethyl-rapamycin, or 40-*O*-tetrazole-rapamycin, 40-epi-(N1-tetrazolyl)-rapamycin, everolimus, biolimus, perfenidone and derivatives, analogs, prodrugs, co-drugs and combinations of any of the foregoing.

Examples of anti-inflammatory agents include both steroidal and non-steroidal (NSAID) anti-inflammatory agents such as, without limitation, clobetasol, alclofenac, alclometasone dipropionate, algestone acetonide, alpha amylase, amcinafal, amcinafide, amfenac sodium, amiprilose hydrochloride, anakinra, anirolac, anitrazafen, apazone, balsalazide disodium, bendazac, benoxaprofen, benzydamine hydrochloride, bromelains, broperamole, budesonide, carprofen, cicloprofen, cintazone, cliprofen, clobetasol propionate, clobetasone butyrate, clopirac, cloticasone propionate, cormethasone acetate, cortodoxone, deflazacore, desonide, desoximetasone, dexamethasone dipropionate, diclofenac potassium, diclofenac sodium, diflorasone diacetate, diflumidone sodium, diflunisal, difluprednate, diftalone, dimethyl sulfoxide, drocinonide, endrysone, enlimomab, enolicam sodium, epirizole, etodolac, etofenamate, felbinac, fenamole, fenbufen, fenclofenac, fenclorac, fendosal, fenpipalone, fentiazac, flazalone, fluazacort, flufenamic acid, flumizole, flunisolide acetate, flunixin, flunixin meglumine, fluocortin butyl, fluorometholone acetate, fluquazone, flurbiprofen, fluretofen, fluticasone propionate, furaprofen, furobufen, halcinonide, halobetasol propionate, halopredone acetate, ibufenac, ibuprofen, ibuprofen aluminum, ibuprofen piconol, ilonidap, indomethacin, indomethacin sodium, indoprofen, indoxole, intrazole, isoflupredone acetate, isoxepac, isoxicam, ketoprofen, lofemizole hydrochloride, lomoxicam, loteprednol etabonate, meclofenamate sodium, meclofenamic acid, meclorisone dibutyrate, mefenamic acid, mesalamine, meseclazone, methylprednisolone suleptanate, momiflumate, nabumetone, naproxen, naproxen sodium, naproxol, nimazone, olsalazine sodium, orgotein, orpanoxin, oxaprozin, oxyphenbutazone, paranyline hydrochloride, pentosan polysulfate sodium, phenbutazone sodium glycerate, pirfenidone, piroxicam, piroxicam cinnamate, piroxicam olamine, pirprofen, prednazate, prifelone, prodolic acid, proquazone, proxazole, proxazole citrate, rimexolone, romazarit, salcolex, salnacedin, salsalate, sanguinarium chloride, seclazone, sermetacin, sudoxicam, sulindac, suprofen, talmetacin, talniflumate, talosalate, tebufelone, tenidap, tenidap sodium, tenoxicam, tesicam, tesimide, tetrydamine, tiopinac, tixocortol pivalate, tolmetin, tolmetin sodium, triclonide, triflumidate, zidometacin, zomepirac sodium, aspirin (acetylsalicylic acid), salicylic acid, corticosteroids, glucocorticoids, tacrolimus, pimecrolimus and derivatives, analogs, prodrugs, co-drugs and combinations of any of the foregoing.

Examples of antineoplastics and antimitotics include, without limitation, paclitaxel, docetaxel, methotrexate, azathioprine, vincristine, vinblastine, fluorouracil, doxorubicin hydrochloride, and mitomycin.

Examples of antiplatelet, anticoagulant, antifibrin, and antithrombin drugs include, without limitation, sodium heparin, low molecular weight heparins, heparinoids, hirudin, argatroban, forskolin, vapiprost, prostacyclin, prostacyclin dextran, D- phe-pro-arg-chloromethylketone, dipyridamole, glycoprotein IIb/IIIa platelet membrane receptor antagonist antibody, recombinant hirudin and thrombin, thrombin inhibitors such as Angiomax ä, calcium channel blockers such as nifedipine, colchicine, fish oil (omega 3-fatty acid), histamine antagonists, lovastatin, monoclonal antibodies such as those specific for Platelet-Derived Growth Factor (PDGF) receptors, nitroprusside, phosphodiesterase inhibitors, prostaglandin inhibitors, suramin, serotonin blockers, steroids, thioprotease inhibitors, triazolopyrimidine, nitric oxide or nitric oxide donors, super oxide dismutases, super oxide dismutase mimetic, 4-amino-2,2,6,6-tetramethylpiperidine-1-oxyl (4-amino-TEMPO) and derivatives, analogs, prodrugs, codrugs and combinations thereof.

Examples of cytostatic or antiproliferative agents include, without limitation, angiopeptin, angiotensin converting enzyme inhibitors such as captopril, cilazapril or lisinopril, calcium channel blockers such as nifedipine; colchicine, fibroblast growth factor (FGF) antagonists; fish oil (ω-3-fatty acid); histamine antagonists; lovastatin, monoclonal antibodies such as, without limitation, those specific for Platelet-Derived Growth Factor (PDGF) receptors; nitroprusside, phosphodiesterase inhibitors, prostaglandin inhibitors, suramin, serotonin blockers, steroids, thioprotease inhibitors, triazolopyrimidine (a PDGF antagonist) and nitric oxide.

Examples of antiallergic agents include, without limitation, permirolast potassium.

Other compounds that may be used as bioactive agents of this invention include, without limitation, alpha-interferon, genetically engineered epithelial cells, dexamethasone, antisense molecules which bind to complementary DNA to inhibit transcription, and ribozymes, antibodies, receptor ligands, enzymes, adhesion peptides, blood clotting factors, inhibitors or clot dissolving agents such as streptokinase and tissue plasminogen activator, antigens for immunization, hormones and growth factors, oligonucleotides such as antisense oligonucleotides and ribozymes and retroviral vectors for use in gene therapy; antiviral agents; analgesics and analgesic combinations; anorexics; antihelmintics; antiarthritics, antiasthmatic agents; anticonvulsants; antidepressants; antidiuretic agents; antidiarrheals; antihistamines; antimigrain preparations; antinauseants; antiparkinsonism drugs; antipruritics; antipsychotics; antipyretics; antispasmodics; anticholinergics; sympathomimetics; xanthine derivatives; cardiovascular preparations including calcium channel blockers and beta-blockers such as pindolol and antiarrhythmics; antihypertensives; diuretics; vasodilators including general coronary; peripheral and cerebral; central nervous system stimulants; cough and cold preparations, including decongestants; hypnotics; immunosuppressives; muscle relaxants; parasympatholytics; psychostimulants; sedatives; tranquilizers; naturally derived or genetically engineered lipoproteins; and derivatives, analogs, prodrugs, codrugs and combinations of any of the foregoing.

Other bioactive agents include a corticosteroid, everolimus, zotarolimus, sirolimus, and derivatives thereof, paclitaxel, biolimus A9, a bisphosphonate, ApoA1, a mutated ApoA1, ApoA1 milano, an ApoA1 mimetic peptide, an ABC A1 agonist, an anti-inflammatory agent, an anti-proliferative agent, an anti-angiogenic agent, a matrix metalloproteinase inhibitor and a tissue inhibitor of metalloproteinase.

The present invention relates to an implantable medical device that includes a ring having a first and second end and one or more bioactive agents adhered to a surface of, integrated into the structure of or disposed within pores in the ring. Methods of adhering to a surface of, integrating into the structure of and disposing within pores of an implantable medical device are known to those skilled in the art.

A ring of the invention will necessarily be made of a material that has the ability to be compressed and to subsequently expand to its original shape. Exemplary ring materials include nitinol, biodegradable materials, polymeric materials and any combination thereof. A presently preferred ring material includes a polymeric material with a nitinol core. The polymeric material will allow for efficient bioactive agent loading and subsequent release while the nitinol core will allow for efficient compression and subsequent expansion of the ring.

Polymeric materials of the invention will be generally biocompatible and can be biodegradable or biostable as well as hydrophobic or hydrophilic.

As used herein, "biocompatible" refers to a polymer that both in its intact, as synthesized state and in its decomposed state, i.e., its degradation products, is not, or at least is minimally, toxic to living tissue; does not, or at least minimally and reparably, injure(s) living tissue; and/or does not, or at least minimally and/or controllably, cause(s) an immunological reaction in living tissue.

Biocompatible, biostable polymers include, without limitation, parylene, poly(D,L-lactide-co-glycolide), poly(1-lactide-co-glycolide) poly(3-hydroxybutyrate), poly(4-hydroxybutyrate), poly(3-hydroxybutyrate-co-3-hydroxyvalerate), polyorthoester, polyanhydride, poly(glycolic acid), poly(glycolide), poly(L-lactic acid), poly(L-lactide), poly(D,L-lactic acid), poly(D,L-lactide), poly(L-lactide-co-D,L-lactide), poly(caprolactone), poly(L-lactide-co-caprolactone), poly(D,L-lactide-co-caprolactone), poly(glycolide-co-caprolactone), poly(trimethylene carbonate), polyester amide, poly(glycolic acid-co-trimethylene carbonate), co-poly(ether-esters) (e.g. PEO/PLA), polyphosphazenes, polyurethanes, silicones, polyesters, polyolefins, polyisobutylene and ethylene-alphaolefin copolymers, acrylic polymers and copolymers other than polyacrylates, vinyl halide polymers and copolymers (such as polyvinyl chloride), polyvinyl ethers (such as polyvinyl methyl ether), polyvinylidene halides (such as polyvinylidene chloride), poly(vinylidene fluoride), poly(vinylidene fluoride-co-hexafluoropropylene), polyacrylonitrile, polyvinyl ketones, polyvinyl aromatics (such as polystyrene), polyvinyl esters (such as polyvinyl acetate), acrylonitrile-styrene copolymers, ABS resins, polyamides (such as Nylon 66 and polycaprolactam), polycarbonates including tyrosine-based polycarbonates, polyoxymethylenes, polyimides, polyethers, polyurethanes, rayon, rayon-triacetate, fullerenes and lipids.

Biocompatible, biodegradable polymers include, without limitation, naturally-occurring polymers such as, without limitation, collagen, chitosan, alginate, fibrin, fibrinogen, cellulosics, starches, dextran, dextrin, hyaluronic acid, heparin, glycosaminoglycans, polysaccharides and elastin.

Synthetic biocompatible, biodegradable polymers include, without limitation, polylactic acid, polyglycolic acid, polyethylene glycol, polycaprolactone, polyanhydrides, polyvinyl alcohol and poly(ester-amides).

As used herein, a synthetic polymer refers to one that is created wholly in the laboratory while a semi-synthetic polymer refers to a naturally-occurring polymer than has been chemically modified in the laboratory. Examples of synthetic polymers include, without limitation, polyphosphazines, polyphosphoesters, polyphosphoester urethane, polyhydroxyacids, polyhydroxyalkanoates, polyanhydrides, polyesters, polyorthoesters, polyamino acids, polyoxymethylenes, poly(ester-amides) and polyimides.

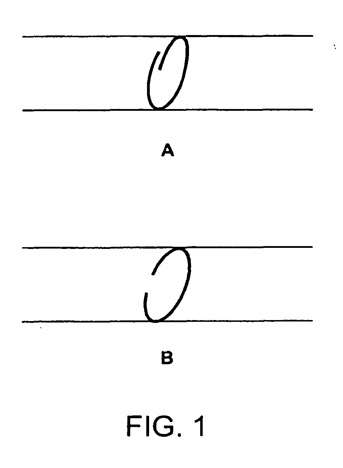

A ring of the invention will also necessarily have an open structure so that it can effectively be compressed when positioned within a catheter or within a casing of the invention as well as expand to the circumference of a vessel after it is deployed Figures 1A-B illustrate rings of the invention in their deployed state. Figure 1A illustrates an open ring with overlapping ends positioned in a blood vessel while Figure 1B illustrates an open ring with ends that do not overlap positioned in a blood vessel. In each situation, the self-expanding material from which the rings are made allow the ring to naturally expand to fit any vessel circumference.

Furthermore, there is disclosed a method for treating or preventing a disease using a bioactive agent-containing ring of the invention. The method involves compressing a ring and positioning it at the distal end of a catheter, inserting the catheter into a blood vessel of a patient upstream of a disease site locale and deploying the ring into the bloodstream of the patient, wherein the ring expands to the circumference of the blood vessel in which it is positioned.

A method of deploying the ring not covered by the claims involves using a deployment plunger having a longitudinally-oriented cylindrical member on which the compressed implantable medical device is positioned.

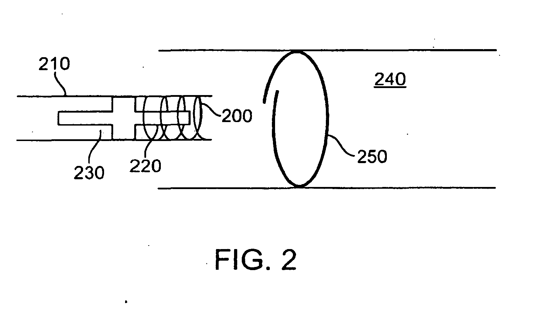

Figure 2 illustrates this method. Ring **200** is compressed within catheter **210** and positioned over longitudinally-oriented cylindrical member **220** of deployment plunger **230.** Catheter **210** is inserted into blood vessel **240** then ring **200** is deployed by plunger **230** into vessel **240** where it expands to the circumference of vessel **240.** Figure 2 also shows a ring **250** in its expanded state positioned along the circumference of vessel **240,** thereby not impeding the flow of blood through vessel **240.** It is to be understood that ring **200** can be deployed in either a 'downstream' manner, for example in the direction of blood flow, or in an 'upstream' manner depending on the needs of the practitioner.

Once the ring is deployed, bioactive agent that was adhered to a surface of, integrated into the structure of or disposed within pores in the ring will diffuse into the vessel wall or into the bloodstream, thereby providing a means for the regional treatment of disease. In specific aspects, the disease to be treated is a vascular disease including, but not limited to, atherosclerosis, restenosis, vulnerable plaque or peripheral arterial disease.

While vascular diseases are presently preferred targets of the composition and methods herein, a host of other diseases are expected to be amenable to treatment or prevention such as, without limitation, cancer, coronary artery disease, kidney disease, liver disease, respiratory disease, infectious diseases and autoimmune diseases.

Cancer refers to malignant neoplasms, which, in turn relate to a large group of diseases that can arise in virtually any tissue composed of potentially dividing cells. The basic characteristic of cancer is a transmissible abnormality of cells that is manifested by reduced control over growth and function leading to serious life-threatening effects on the host through invasive growth and metastases.

Coronary artery disease refers to a narrowing of the coronary arteries cause by atherosclerosis that, when sufficiently severe, limits, or, in its most serious form completely occludes, the flow of blood to the myocardium (heart muscle).

Respiratory disease refers to a disease in which the lungs do not work properly so that breathing is affected. Examples of respiratory diseases include, without limitation, asthma, tuberculosis, cystic fibrosis and pneumonia.

Kidney disease refers to any disease or disorder that affects the function of the kidneys such as, without limitation, acute nephritic syndrome, atheroembolic renal disease, chronic nephritis, kidney cancer, Goodpasture's syndrome, interstitial nephritis and lupus nephritis.

Liver disease refers to any disease that affects the function of the liver such as, without limitation, hepatitis (A, B, C, D or autoimmune), liver cancer and cirrhosis.

Infectious disease refers to any disease transmitted by a microorganism such as, without limitation, a bacterium, a virus, a prion, a fungus, an amoeba or a protozoon. In general, infectious diseases are communicable in nature and may be transmitted from one individual to another and are capable of producing serious illness in the other individual.

Autoimmune disease refers to diseases and disorders (and related diseases and disorders) involving an abnormal immune response such as, without limitation, Hashimoto's thyroiditis, diabetes, rheumatoid arthritis, systemic lupus erythematosus, Sjogren syndrome, multiple sclerosis, myasthenia gravis and Grave's disease.

In another aspect of the invention, a ring of the invention is compressed and encased in a degradable casing. Suitable casing materials include biodegradable, biosoluble and biocompatible polymeric materials and are described above. Methods of encasing a device are known to those skilled in the art. In this aspect of the invention, the degradable casing can also contain a spherical member, which can be biodegradable or biostable, to which the distal end of a tether is directly attached. Suitable biodegradable and biostable materials are described above. In this aspect of the invention, the degradable casing can also be adjacent to a spherical member.

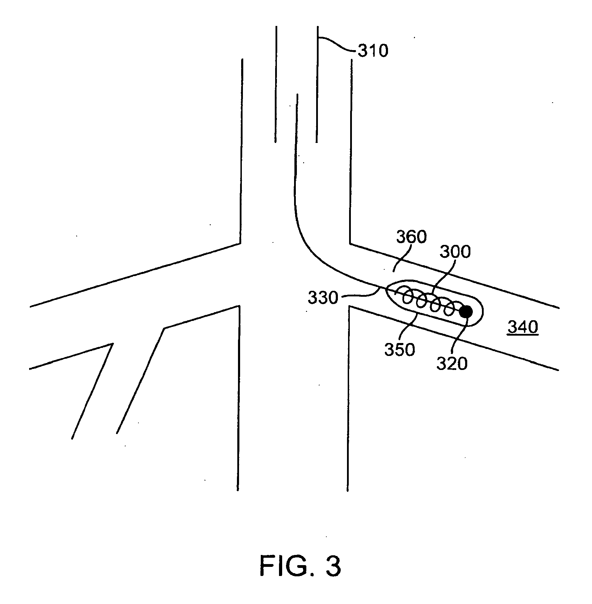

Figure 3 illustrates this aspect of the invention. Spherical member 320 is connected to the distal end of tether **330** inside of casing **350.** Ring **300** encircles tether **330** which, when deployed *in vivo,* runs from spherical member **320** through the vasculature to catheter **310.** Spherical member **320** acts to orient ring **300** so that upon casing **350** degradation ring **300** will be positioned along the circumference of vessel **340.**

The disclosure yet further relates to a method for treating or preventing a disease using a ring encased in a biodegradable casing as described above. The method involves positioning the encased ring, i.e., device, in the distal end of a catheter, inserting the catheter into a blood vessel of a patient upstream of a disease site locale and deploying the device into the bloodstream of the patient such that the device is allowed to move in the direction of blood flow. Using the attached tether, the encased ring can be positioned at or near a disease site locale while the proximal end of the tether remains operatively coupled to the catheter. Figure 3 illustrates one embodiment of this aspect. It is to be understood that degradable casing materials will be chosen so as to allow sufficient time for the device to become positioned upstream of a disease site locale but degrade quickly enough so as to not block blood flow for any extended amount of time.

Once encased ring **360** is positioned upstream of a disease site locale, casing **350** will degrade. During this time ring **300** which abuts spherical member **320** will begin to slowly expand as casing **350** degrades. Once casing **350** has completely degraded, ring **300** will expand fully to match the circumference of blood vessel **340.** Spherical member **320** will then be able to be pulled back into catheter **310** by tether **330** or will degrade on its own. In either case, tether **330** will be pulled back into catheter **310.**

While vascular diseases are presently preferred targets of these methods, other diseases are expected to be amenable to treatment or prevention and are described above.

It is to be understood that multiple rings and multiple tethers can be used concurrently with any of the above aspects of the invention and are encompassed by the present invention. Indeed, in various aspects, the implantable medical device includes a plurality of tethers wherein one open ring-shaped device encircles each tether and abuts a spherical member. In other aspects, the implantable medical device includes two or more open-ringed shaped devices encircling at least one tether. In this aspect, the two or more open-ringed shaped devices can each individually abut a spherical member coupled to the tether thereby forming a 'beads on a string' sort of formation.

When multiple rings are used in a single casing, the segments may be open-ended such that the ring edges are overlapping. Alternatively, the rings may be shaped so as to separate from each other upon expansion.

While particular embodiments of the present invention have been shown and described, it will be obvious to those skilled in the art that changes and modifications can be made without departing from this invention in its broader aspects. Therefore, the claims are to encompass within their scope all such changes and modifications.

## Claims

1. An implantable medical device comprising:
an open ring-shaped device body (300) having a first and second end; and
one or more bioactive agents adhered to a surface of, integrated into the structure of or disposed within pores in the device body, **characterized by**
further comprising a tether (330),
wherein the open ring-shaped device body (300) encircles the tether.

2. The implantable medical device according to claim 1, wherein the first and second ends of the open ring-shaped device body overlap each other.

3. The implantable medical device according to claim 1, wherein the first and second ends of the open ring-shaped device body do not overlap each other.

4. The implantable medical device according to claim 1, wherein the device body comprises a material selected from the group consisting of nitinol, a biodegradable material, a polymeric material or any combination thereof.

5. The implantable medical device according to claim 4, wherein the device body comprises a polymeric material and a nitinol core.

6. The implantable medical device according to claim 1, wherein the implantable medical device further comprises two or more open ring-shaped device bodies.

7. The implantable medical device according to claim 1, wherein the implantable medical device further comprises a degradable casing in which the open ring-shaped device body is contained in a compressed state.

8. The implantable medical device according to claim 7, wherein the casing degrades within 10 seconds to 10 minutes.

9. The implantable medical device according to claim 1, wherein the open ring-shaped body (300) abuts a spherical member (320) coupled to the distal end of the tether.

10. The implantable medical device according to claim 9, wherein the spherical member (320) is biodegradable.

11. The implantable medical device according to claim 1, further comprising a plurality of tethers wherein one open ring-shaped device encircles each tether and abuts a spherical member (320).

12. The implantable medical device according to claim 11, further comprising two or more open ring-shaped devices encircling at least one tether.

13. The implantable medical device according to claim 12, wherein the two or more open ring-shaped devices each individually abut a spherical member (320) coupled to the tether.

14. The implantable medical device according to any one of claims 9 to 13, further comprising a degradable casing (350) in which the open ring-shaped device body is contained in a compressed state.

## Patentansprüche

1. Eine implantierbare medizinische Vorrichtung umfassend:
einen offenen ringförmigen Körper (300), der ein erstes Ende und ein zweites Ende aufweist; und
ein oder mehrere bioaktive Mittel, die entweder auf der Oberfläche des Körpers befestigt, in seiner Struktur integriert oder innerhalb von Poren im Körper angebracht sind, **dadurch gekennzeichnet, dass**
die Vorrichtung außerdem eine Halteleine (330) umfasst,
wobei der offene ringförmige Körper (300) die Halteleine umschließt.

2. Die implantierbare medizinische Vorrichtung nach Anspruch 1, wobei das erste Ende und das zweite Ende des offenen ringförmigen Körpers sich gegenseitig überlagern.

3. Die implantierbare medizinische Vorrichtung nach Anspruch 1, wobei das erste Ende und das zweite Ende des offenen ringförmigen Körpers sich nicht gegenseitig überlagern.

4. Die implantierbare medizinische Vorrichtung nach Anspruch 1, wobei der Körper ein Material umfasst, das aus der Gruppe bestehend aus Nitinol, einem biologisch abbaubaren Material, einem polymerischen Material oder einer beliebigen Kombination dieser Materialien ausgewählt wurde.

5. Die implantierbare medizinische Vorrichtung nach Anspruch 4, wobei der Körper ein polymerisches Material und einen Kern aus Nitinol umfasst.

6. Die implantierbare medizinische Vorrichtung nach Anspruch 1, wobei die implantierbare medizinische Vorrichtung zusätzlich zwei oder mehrere offene ringförmige Körper umfasst.

7. Die implantierbare medizinische Vorrichtung nach Anspruch 1, wobei die implantierbare medizinische Vorrichtung zusätzlich ein abbaubares Gehäuse umfasst, in welchem der offene ringförmige Körper in einem komprimierten Zustand enthalten ist.

8. Die implantierbare medizinische Vorrichtung nach Anspruch 7, wobei das Gehäuse sich in einem Zeitraum zwischen 10 Sekunden und 10 Minuten abbaut.

9. Die implantierbare medizinische Vorrichtung nach Anspruch 1, wobei der offene ringförmige Körper (300) an ein kugelförmiges Bauteil (320) anstößt, das an das distale Ende der Halteleine angekoppelt ist.

10. Die implantierbare medizinische Vorrichtung nach Anspruch 9, wobei das kugelförmiges Bauteil (320) biologisch abbaubar ist.

11. Die implantierbare medizinische Vorrichtung nach Anspruch 1, wobei die Vorrichtung zusätzlich eine Vielzahl an Halteleinen umfasst, wobei jede Halteleine von einem offenen ringförmigen Körper umfasst wird, der an ein kugelförmiges Bauteil (320) anstößt.

12. Die implantierbare medizinische Vorrichtung nach Anspruch 11, wobei die Vorrichtung zusätzlich zwei oder mehr offene ringförmige Körper umfasst, die mindestens eine Halteleine umschließen.

13. Die implantierbare medizinische Vorrichtung nach Anspruch 12, wobei jeder einzelne der zwei oder mehr offenen ringförmigen Körper an ein kugelförmiges, an die Halteleine gekoppeltes Bauteil (320) anstößt.

14. Die implantierbare medizinische Vorrichtung nach einem der Ansprüche 9 bis 13, wobei die Vorrichtung zusätzlich ein abbaubares Gehäuse (350) umfasst, in welchem der offene ringförmige Körper in einem komprimierten Zustand enthalten ist.

## Revendications

1. Dispositif médical implantable comprenant :
un corps de dispositif en forme d'anneau ouvert (300) ayant une première et une seconde extrémités ; et
un ou plusieurs agents bioactifs mis en adhésion sur une surface du corps de dispositif, intégré dans la structure de celui-ci, ou disposé au sein de pores dans celui-ci,
**caractérisé en ce qu'**il comprend en outre un câble d'attache (330),
le corps de dispositif en forme d'anneau ouvert (300) encerclant le câble d'attache.

2. Dispositif médical implantable selon la revendication 1, dans lequel les première et seconde extrémités du corps de dispositif en forme d'anneau ouvert se chevauchent mutuellement.

3. Dispositif médical implantable selon la revendication 1, dans lequel les première et seconde extrémités du corps de dispositif en forme d'anneau ouvert ne se chevauchent pas mutuellement.

4. Dispositif médical implantable selon la revendication 1, dans lequel le corps de dispositif comprend un matériau choisi dans le groupe constitué du nitinol, un matériau biodégradable, un matériau polymère ou n'importe quelle combinaison de ceux-ci.

5. Dispositif médical implantable selon la revendication 4, dans lequel le corps de dispositif comprend un matériau polymère et un coeur en nitinol.

6. Dispositif médical implantable selon la revendication 1, où le dispositif médical implantable comprend en outre deux ou plusieurs corps de dispositif en forme d'anneau ouvert.

7. Dispositif médical implantable selon la revendication 1, où le dispositif médical implantable comprend en outre un boîtier dégradable dans lequel le corps de dispositif en forme d'anneau ouvert est contenu dans un état comprimé.

8. Dispositif médical implantable selon la revendication 7, dans lequel le boîtier se dégrade endéans 10 secondes à 10 minutes.

9. Dispositif médical implantable selon la revendication 1, dans lequel le corps en forme d'anneau ouvert (300) vient en butée contre un membre sphérique (320) couplé à l'extrémité distale du câble d'attache.

10. Dispositif médical implantable selon la revendication 9, dans lequel le membre sphérique (320) est biodégradable.

11. Dispositif médical implantable selon la revendication 1, comprenant en outre une pluralité de câbles d'attache, chaque câble d'attache étant encerclé par un dispositif en forme d'anneau ouvert qui vient en butée contre un membre sphérique (320).

12. Dispositif médical implantable selon la revendication 11, comprenant en outre deux dispositifs en forme d'anneau ouvert ou plus encerclant au moins un câble d'attache.

13. Dispositif médical implantable selon la revendication 12, dans lequel les deux dispositifs en forme d'anneau ouvert ou plus viennent chacun individuellement en butée contre un membre sphérique (320) couplé au câble d'attache.

14. Dispositif médical implantable selon l'une quelconque des revendications 9 à 13, comprenant en outre un boîtier dégradable (350) dans lequel le corps de dispositif en forme d'anneau ouvert est contenu dans un état comprimé.
